# EUROPÄISCHE PATENTANMELDUNG

(11) **EP 2 888 992 A1**
(43) Veröffentlichungstag der Anmeldung: **01.07.2015**
(21) Anmeldenummer: 14197962.5
(22) Anmeldetag: 17.03.2010
(51) Int. Cl.: A61B 1/253

(54) **Medizinisches, insbesondere zahnmedizinisches Diagnosegerät mit Bilderfassungsmitteln**

(30) Priorität: 17.03.2009 DE 102009013615
(62) Teilanmeldung aus: 10711650.1
(71) Anmelder: Kaltenbach & Voigt GmbH, 88400 Biberach (DE)
(72) Erfinder: Erdmann, Sven, 89081 Ulm (DE); Hackel, André, 88400 Biberach (DE)
(74) Vertreter: Thun, Clemens

(57) **Zusammenfassung**

Bei einem zahnmedizinischen Diagnosegerät mit Lichtquellen zur Illumination eines Zahns, Mitteln zur Bilderfassung des illuminierten Zahns sowie einem Handstück (1), welches ein im Strahlengang der Mittel zur Bilderfassung befindliches Fenster (5) aufweist, sind ferner Mittel zur Beheizung des Fensters (5) vorgesehen sind, welche durch eine Widerstandsheizung gebildet sind, wobei das Handstück (1) ferner mindestens einen Arm (4, 7) mit einem daran angeordneten Lichtaustrittselement aufweist.

## Beschreibung

Die vorliegende Erfindung betrifft ein medizinisches, insbesondere ein zahnmedizinisches Diagnosegerät gemäß dem Oberbegriff des Anspruchs 1, welches Mittel zur Bilderfassung sowie ein Handstück mit einem im Strahlengang der Mittel zur Bilderfassung befindliches Fenster aufweist, wobei ferner eine Beheizung des Fensters vorgesehen ist. Insbesondere betrifft die vorliegende Erfindung eine so genannte Intraoralkamera oder ein System zum Transilluminieren von Zähnen.

In der Medizin, insbesondere der Zahnmedizin werden vermehrt Diagnosesysteme eingesetzt, welche auf optischen Prinzipien beruhen. Grund hierfür ist, dass derartige Geräte üblicherweise berührungslos, also insbesondere schmerzfrei die Erstellung einer Diagnose ermöglichen und darüber hinaus auch in der Regel optische Bilder zur Verfügung stellen, anhand welcher evtl. erforderliche therapeutische Maßnahmen dem Patienten besser vermittelt werden können. Hierfür kommen bspw. so genannte Intraoralkameras zum Einsatz, welche ein Handstück beinhalten, dessen vorderer Endbereich in den Mund eines Patienten eingeführt wird. In diesem Endbereich befindet sich in der Regel ein Lichteintritts- bzw. Sichtfenster für die Kameraoptik, von dem ausgehend das Bild des zu untersuchenden Objekts einer Erfassungseinrichtung, bspw. einem CCD-Chip übermittelt wird. Diese klassische Intraoralkamera kann ferner zu einem System zum Transilluminieren von Zähnen erweitert werden, wie es bspw. aus der DE 10 2006 041 020 A1 der Anmelderin bekannt ist. Hierbei wird der zu untersuchende Zahn mit sichtbarem Licht bestrahlt, wobei dann ein optisches Bild des durch die Untersuchungsstrahlung illuminierten Zahns aufgenommen und bewertet wird. Da kariöse Stellen im Zahn das Licht anders streuen als gesundes Zahngewebe, können derartige Stellen bei Erfassung des Zahns mit Hilfe einer Kamera identifiziert werden.

In beiden Fällen besteht das Problem, dass die Qualität des erhaltenen Bildes nur dann zufriedenstellend ist, wenn das Sichtfenster im vorderen Bereich des Handstücks einen ungestörten Lichteintritt zulässt. Aufgrund der Temperaturunterschiede innerhalb und außerhalb des Mundraums eines Patienten besteht allerdings bei derartigen intraoralen Kameras oftmals das Problem, dass das Fenster nach Einführen der Kamera in den Mundraum beschlägt. Eine vergleichbare Problematik ergibt sich auch in anderen medizinischen Bereichen, bei denen bspw. mit Hilfe eines Katheters optische Aufnahmen aus dem Innenbereich eines zu untersuchenden Patienten erstellt werden sollen.

Um die Problematik des Beschlagens des Fensters für die Kamera zu vermeiden, wurde bereits vorgeschlagen, das Fenster aufzuheizen. Bislang bekannte Lösungen hierfür basieren auf der Wärmekopplung innenliegender Lichtquellen mit dem Fenster bzw. dem gezielten Aufheizen der Kamera in externen Ablageeinrichtungen.

Die Wärmekopplung innenliegender Lichtquellen an das Fenster wurde bspw. mit dem Einsatz von LEDs und der damit verbundenen nahen Platzierung zum Kamerafenster ermöglicht. LEDs produzieren bekanntlicherweise einen nicht unerheblichen Anteil von Verlustwärme, welche dann über die sich hieraus ergebende Wärmestrahlung für das Aufheizen des Fensters genutzt werden kann. Nachteilig an dieser Lösung ist allerdings, dass die Plazierung der Beleuchtungsmittel am distalen Ende des Handstücks zu einer Vergrößerung des Instruments führt, was generell aufgrund der räumlich begrenzten Mundhöhle nicht erwünscht ist. Ferner bietet diese Lösung keinerlei Möglichkeiten, die Erwärmung des Fensters bei unterschiedlichen Umgebungstemperaturen zu regeln.

Bei einer Aufheizung des Kamerahandstücks in einer externen Ablage ergibt sich der Nachteil, dass das Handstück - um die Betriebsbereitschaft dauerhaft aufrecht zu erhalten - grundsätzlich in dieser Ablage angeordnet werden sollte. Ferner ist diese Lösung mit dem Erfordernis einer zusätzlichen Stromzuführung verbunden.

Der vorliegenden Erfindung liegt deshalb die Aufgabe zugrunde, eine neuartige Lösung zur Vermeidung der oben genannten Probleme anzugeben. Insbesondere soll die Möglichkeit eröffnet werden, das Sichtfenster eines medizinischen bzw. zahnmedizinischen Diagnosegeräts, welches Bilderfassungsmittel aufweist, einfach und effektiv aufzuheizen.

Die Aufgabe wird durch ein medizinisches bzw. zahnmedizinisches Diagnosegerät, welches die Merkmale des Anspruchs 1 aufweist, gelöst. Vorteilhafte Weiterbildungen der Erfindung sind Gegenstand der abhängigen Ansprüche.

Die erfindungsgemäße Lösung beruht auf der Idee, das Kamerafenster mit Hilfe einer Widerstandsheizung zu erwärmen.

Erfindungsgemäß wird dementsprechend ein medizinisches, insbesondere ein zahnmedizinisches Diagnosegerät mit Mitteln zur Bilderfassung sowie einem Handstück, welches ein im Strahlengang der Mittel zur Bilderfassung befindliches Fenster aufweist, vorgeschlagen, wobei ferner Mittel zur Beheizung des Fensters vorgesehen sind und die Mittel zur Beheizung des Fensters durch eine Widerstandsheizung gebildet sind.

Die erfindungsgemäße Lösung zeichnet sich dadurch aus, dass sie eine Verlagerung der Beleuchtungskörper in Richtung des proximalen Endes des Handstücks bei Realisierung einer eigenständigen Fensterheizung ermöglicht. Hierdurch kann der Kopfbereich des Handgeräts besonders kompakt gestaltet werden, was im Hinblick auf die Handhabung des Geräts und der Durchführung von Untersuchungen deutliche Vorteile mit sich bringt. Ferner führt die erfindungsgemäße Lösung zu einer äußerst genauen und zuverlässigen Beheizung des Fensters, was während des Betriebs zu zusätzlichen Vorteilen führt.

Gemäß einer vorteilhaften Weiterbildung der Erfindung kann die Widerstandsheizung eine Leiterbahnanordnung aufweisen, welche bspw. unmittelbar auf einem das Fenster bildenden optisch transparenten Substrat angeordnet ist. Die Leiterbahnanordnung könnte allerdings auch auf einem Trägerelement angeordnet sein, welches mit dem das Fenster bildenden Substrat gekoppelt ist. Bei diesem Trägerelement kann es sich bspw. um eine herkömmliche Leiterplatte, um ein sogenanntes MID (Moulded Interconnected Device) als Zweikomponenten-Kunststoffspritzgußteil bzw. ein MID als Einkomponenten-Kunststoffspritzgußteil, welches im LDS-(Laser-Direkt-Strukturierung) Verfahren hergestellt wurde, handeln. Das Trägerelement kann dabei zusätzliche weitere elektronische Bauteile aufweisen, bspw. Widerstände oder Temperaturmesselemente. Kommen Temperaturmesselemente zum Einsatz, so umfassen die Mittel zur Beheizung des Fensters vorzugsweise auch eine Steuereinheit, welche dazu ausgebildet ist, einen durch die Leiterbahnanordnung fließenden Strom zu regeln. Auf diese Weise kann eine zuverlässige Erwärmung des Kamerafensters innerhalb eines gewünschten Temperaturbereichs erzielt werden.

Alternativ hierzu bestünde auch die Möglichkeit, ein so genanntes PTC-Element (Positiv Temperature Coefficient) zu verwenden. Dieses oftmals auch als Kaltleiterelement bezeichnete Element kann dann derart gewählt werden, dass eine selbstregelnde Heizung erzielt wird und insbesondere die geforderte Maximaltemperatur des Kamerafensters zur Vermeidung thermischer Irritationen und Verbrennungen nicht überschritten wird. In diesem Fall kann also auf zusätzliche Temperatursensoren zur Erfassung der tatsächlichen Fenstertemperatur und ggf. Abschalten des Heizkreises verzichtet werden. Schließlich bestünde auch die Möglichkeit, zum Beheizen des Fensters einen in der Nähe des Fensters befindlichen Widerstandsdraht einzusetzen.

Die Mittel zur Beheizung des Fensters sind vorzugsweise ummantelt bzw. in ein isolierendes Material integriert, um die geforderten Kriech- und Luftstrecken einzuhalten. Eine Gefährdung des Patienten während der Benutzung des Geräts kann auf diesem Wege ausgeschlossen werden.

Bei dem erfindungsgemäßen Diagnosegerät kann es sich insbesondere um eine Intraoralkamera oder um ein Gerät handeln, welches Bestandteil eines Systems zum Transilluminieren von Zähnen ist. Das Konzept der erfindungsgemäßen Fensterbeheizung ist allerdings auch auf andere medizinische Diagnosegeräte, bspw. Katheter, Endoskope und dergleichen anwendbar.

Nachfolgend soll die Erfindung anhand der beiliegenden Zeichnung näher erläutert werden. Es zeigen:
- Fig. 1a und 1b: Ansichten des vorderen Bereichs eines medizinischen Handstücks, welches Bestandteil eines Systems zum Transilluminieren von Zähnen ist;
- Fig. 2: schematisch die Ausgestaltung eines ersten Ausführungsbeispiels einer erfindungsgemäßen Vorrichtung zum Beheizen des Kamerafensters;
- Fig. 3 bis 5: weitere Ansichten zur näheren Ausgestaltung der Heizung gemäß der in Fig. 2 dargestellten Variante;
- Fig. 6: schematisch ein zweites Ausführungsbeispiel einer erfindungsgemäßen Fensterheizung;
- Fig. 7 bis 10: einen zur Heizung des Kamerafensters genutzten Widerstandsdraht;
- Fig. 11 und 12: eine erste Variante zum Beheizen des Kamerafensters mittels einer sog. Heatpipe; und
- Fig. 13: eine zweite Variante zum Beheizen des Kamerafensters mittels einer Heatpipe.

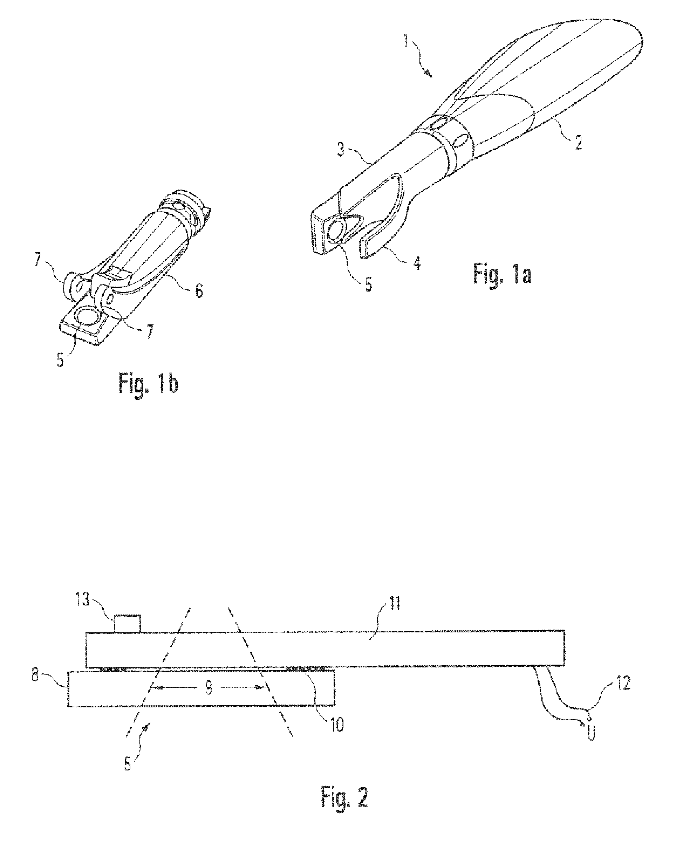

Die Fig. 1a und 1b zeigen als denkbares Anwendungsbeispiel der vorliegenden Erfindung den vorderen Endbereich eines Handstücks, welches in einem System zum Transilluminieren von Zähnen verwendet wird. Das mit dem Bezugszeichen 1 versehene Handstück weist eine längliche Griffhülse 2 auf, an deren vorderen Ende ein Aufsatz 3 angeordnet ist. Mit Hilfe dieses Aufsatzes 3 wird einerseits ein zu untersuchender Zahn (nicht dargestellt) mit Licht bestrahlt, andererseits wird das hierbei entstehende Bild des illuminierten Zahns aufgenommen und ausgewertet.

Die Untersuchungsstrahlung liegt üblicherweise in einem Wellenlängenbereich von etwa 550 nm bis 790 nm, z. Bsp. bei etwa 670 nm. Das Prinzip dieser optischen Untersuchung basiert darauf, dass das Gewebe des Zahns die Untersuchungsstrahlung nicht vollständig abblockt, sondern stattdessen erlaubt, dass die Strahlung durch den Zahn hindurchtritt. Hierbei wird die Strahlung teilweise gestreut, wobei insbesondere kariöse Bereiche für eine charakteristische Beeinflussung des Lichts sorgen. Wird der auf diese Weise transilluminierte Zahn aus verschiedenen Blickrichtungen betrachtet, so können kariöse Bereiche erkannt werden, da diese etwas dunkler erscheinen.

Um die Handhabung des Geräts während der Untersuchung zu erleichtern, sollten die Abmessungen insbesondere des vorderen Endbereichs möglichst gering sein. Dementsprechend sind im dargestellten Beispiel weder die Lichtquellen noch die eigentlichen Mittel zur Bildaufnahme, bspw. ein CCD-Chip in dem Aufsatz 3 selbst angeordnet (wobei die Anwendung des erfindungsgemäßen Prinzips selbstverständlich auch bei einer Anordnung des Chips in dem Aufsatz 3 möglich wäre), sondern befinden sich vielmehr im hinteren Bereich des Handstücks 1. Zur Illumination des Zahns wird dann das von den in dem Handstück 1 angeordneten Lichtquellen emittierte Licht über Lichtleiter in den Aufsatz 3 eingekoppelt und hier über ein Lichtaustrittselement, welches an einem unteren Arm 4 angeordnet ist, abgestrahlt. Gegenüber dem Lichtaustrittselement ist ein üblicherweise kreisförmiges Fenster 5 angeordnet, über welches das Bild des illuminierten Zahns erfasst wird. Das Fenster 5 ist über optische Elemente mit den Bilderfassungsmitteln gekoppelt, wobei hierfür in erster Linie Spiegel, Prismen, Linsen und dergleichen zum Einsatz kommen.

Fig. 1b zeigt eine zweite Ausführungsform eines Aufsatzes 6, wobei hierbei die Bestrahlung des zu untersuchenden Zahns nicht an der dem Fenster 5 gegenüberliegenden Seite stattfindet sondern in einem Winkel hierzu. Der Aufsatz weist hierzu zwei seitliche Arme 7 auf, an deren Endbereichen wieder die Lichtaustrittsenden von Lichtleitern angeordnet sind, die mit den internen Lichtquellen gekoppelt sind.

Bei beiden Varianten kann ein qualitativ hochwertiges Bild nur dann erhalten werden, wenn der Lichteintritt über das Fenster 5 ungestört erfolgt. Hierbei besteht insbesondere die Problematik, dass die unterschiedlichen Temperaturen innerhalb und außerhalb des Mundraums eines Patienten sowie unterschiedliche Luftfeuchtigkeitswerte zu einem Beschlagen des Fensters 5 führen können, was zur Folge hat, dass zumindest vorübergehend keine optischen Aufnahmen erstellt werden können. Um diesen Effekt zu vermeiden, ist vorgesehen, das Fenster 5 aktiv zu beheizen, wobei dies gemäß der vorliegenden Erfindung durch eine Widerstandsheizung erfolgt.

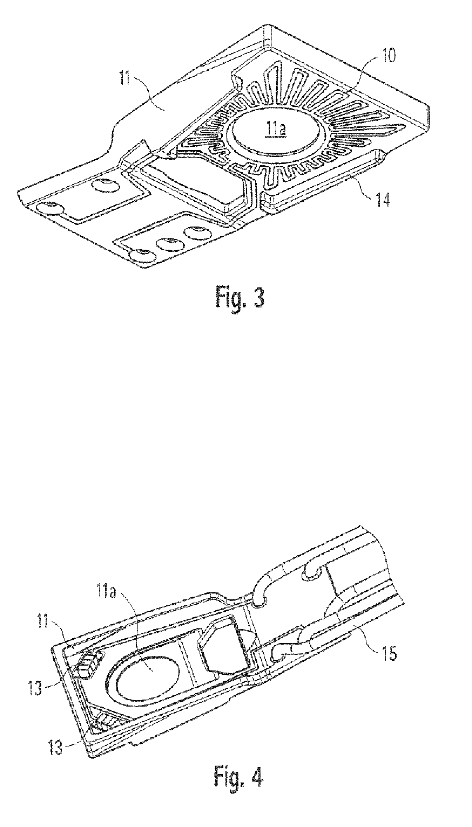

Ein erstes Ausführungsbeispiel des erfindungsgemäßen Konzepts ist in den Fig. 2 bis 5 dargestellt, wobei Fig. 2 schematisch die Ausgestaltung der erfindungsgemäßen Fensterheizung zeigt und die Fig. 3 bis 5 eine konkrete Ausführungsform zeigen.

Die erste Variante der erfindungsgemäßen Lösung beruht auf dem Gedanken, das Fenster 5 unmittelbar über eine Widerstandsheizung zu erwärmen. Hierfür ist eine Anordnung von Leiterbahnen 10 vorgesehen, welche direkt mit dem Fenstersubstrat 8 verbunden und thermisch an dieses angekoppelt sind. Das Fenstersubstrat 8 kann bspw. aus Quarzglas, Saphir, Kunststoff oder einem anderen optisch transparenten Material bestehen. Die Leiterbahnen 2 können unmittelbar auf dem Fenstersubstrat 8 aufgebracht sein, vorzugsweise sind sie jedoch auf der Oberfläche eines Trägerelements 11 angeordnet. Bei diesem Trägerelement 11 kann es sich um eine herkömmliche Leiterplatte oder um ein so genanntes MID (Moulded Interconnected Device) handeln, welches als Zweikomponenten-Kunststoffspritzgussteil oder als Einkomponenten-Kunststoffspritzgußteil ausgebildet sein kann, das im LDS-Verfahren hergestellt wurde. Selbstverständlich wären auch die alternativen Verfahren zum Herstellen eines MID denkbar. Hierbei sind insbesondere das Folienheißprägen, das Folienhinterspritzen, das Folienvorspritzen oder ähnliche Verfahren zu nennen.

Die Leiterbahnen 10 sind derart im Bereich des Fensters 5 angeordnet, dass sie außerhalb des optischen Durchgangs 9 für die Kameraoptik liegen und dementsprechend die Bildübertragung zu den Bilderfassungsmitteln nicht stören. Wird nunmehr über eine schematisch dargestellte Stromversorgungsquelle 12 eine Spannung an die Leiterbahnanordnung 10 angelegt, so führt dies aufgrund des Widerstands der Leiterbahnen 10 zu der Entstehung von Verlustwärme, welche aufgrund der optimierten Anordnung der Leiterbahnen 10 unmittelbar zu einer Erwärmung des Fensters 5 führt. Hierdurch kann ein Beschlagen des Fensters 5 sehr effektiv vermieden werden.

Die dargestellte Variante, bei der die Leiterbahnen 10 auf dem Trägerelement 11 angeordnet sind, eröffnet hierbei die Möglichkeit, zusätzliche elektronische Bauteile, bspw. Widerstände oder dergleichen auf dem Trägerelement 11 anzuordnen und mit den Leiterbahnen 10 zu verbinden. Widerstände werden dabei vorzugsweise wiederum in der Nähe des Fensters 5 angeordnet, um eine zusätzliche Erwähnung zu erzielen.

Eine Temperaturüberwachung erfolgt über ein oder mehrere Temperaturmesselemente 13, welche ebenfalls auf dem Trägerelement 11 in der Nähe des Fensters 5 angeordnet sind. Unter Berücksichtigung der sich zwangsläufig ergebenden Temperaturdifferenz zwischen der dem Fenster 5 zugewandten Seite und der dem Fenster 5 abgewandten Seite kann die Temperatur des Fensters 5 hinreichend genau ermittelt werden. Diese Informationen werden dann von einer nicht näher dargestellten Steuereinheit verarbeitet, welche in entsprechender Weise die Stromversorgungsquelle 12 ansteuert und hierdurch eine genaue Regelung der Fenstertemperatur ermöglicht.

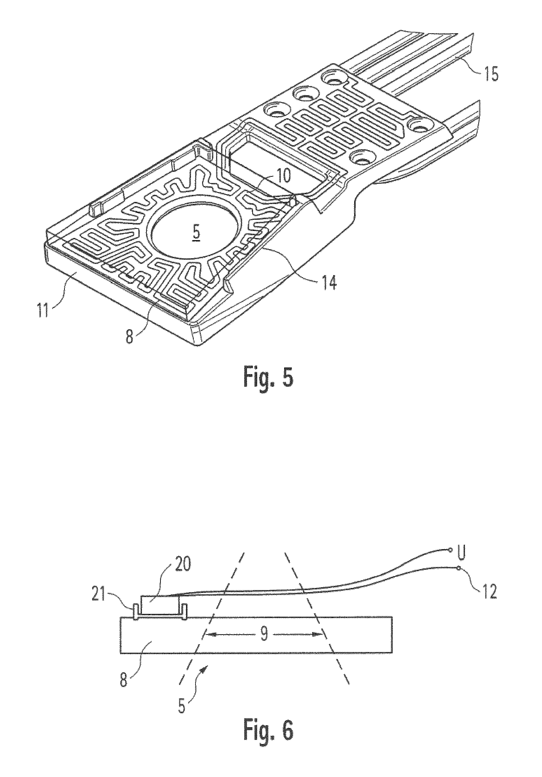

Bei einer Anwendung der erfindungsgemäßen Fensterheizung im medizinischen Bereich sollten selbstverständlich Gefährdungen für den Patienten ausgeschlossen werden, weshalb die gültigen Normen in Bezug auf Luft- und Kriechstrecken eingehalten werden müssen. Dies führt zu einer Bevorzugung von Kunststoffspritzgussverfahren beim Erstellen des Trägerelements 11 mit den Leiterbahnen 10, da hierbei geeignete Abschirmelemente in der Umgebung der Leiterbahnen 10 angebracht werden können. Diese in Fig. 5 dargestellten Abschirmelemente 14 ermöglichen es, die vorgeschriebenen Luft- und Kriechstrecken zu erzielen. Allgemein kann durch eine geeignete Ummantelung der Leiterbahnen 10, welche bei der Verwendung von Kunststoffspritzgussverfahren verhältnismäßig einfach erstellt werden kann, die angestrebte Sicherheit für den Patienten gewährleistet werden.

Die Fig. 3 bis 5 zeigen die konkrete Ausführungsform eines Trägerelements 11, welches in der erfindungsgemäßen Weise ausgestaltet ist, wobei gleiche Elemente mit den gleichen Bezugszeichen versehen wurden.

Das Trägerelement 11 weist hierbei eine Durchgangsöffnung bzw. einen optischen Durchgang 11a auf, der den Lichtdurchtritt durch das Fenster 5 und die Weiterleitung zu den Bilderfassungsmitteln ermöglicht. Erkennbar ist ferner, dass mehrere Litzen 15 zu der Rückseite des Trägerelements 11 führen, wobei über diese Litzen 15 die Stromversorgung der Leiterbahnen 10 erfolgt. Diese sind bevorzugt meanderförmig im Umgebungsbereich des optischen Durchgangs 11a angeordnet, so dass das Fenstersubstrat 8 über seine gesamte Fläche hinweg und damit sehr effektiv beheizt werden kann. Auch die Übermittlung der von den Temperaturmesselementen 13 ausgegebenen Signale zu der Steuereinheit erfolgt über die Litzen 15.

Die erfindungsgemäße Lösung zeichnet sich einerseits dadurch aus, dass das Fenstersubstrat 8 sehr schnell und effektiv beheizt werden kann. Ferner beanspruchen die Mittel zur Heizung des Fensters 5 nur wenig Platz, so dass sie wie dargestellt unmittelbar in der Nähe des Fensters 5 angeordnet werden können, wodurch die Effektivität der Heizung zusätzlich erhöht wird.

Eine weitere Ausführungsform der erfindungsgemäßen Fensterheizung ist in Fig. 6 schematisch dargestellt. Hierbei wird nunmehr die Beheizung des Fenstersubstrats 8 mit Hilfe eines so genannten Kaltleiterelements 20 bzw. PTC-Elements (Positive Temperature Coefficient) erzielt. Wiederum wird durch die Zuführung von Strom zu dem PTC-Element 20 eine Erwärmung erzielt, wobei die Widerstandskennlinie des Kaltleiterelements 20 so gewählt ist, dass das Element 20 selbstregelnd wirkt und dementsprechend die geforderte Maximaltemperatur des Fensters 5 zur Vermeidung thermischer Irritationen und Verbrennungen nicht überschritten wird. Zusätzliche Temperatursensoren zur Erfassung der tatsächlichen Fenstertemperatur und Abschaltung des Heizkreises sind in diesem Fall nicht erforderlich.

Je nach Ausführung des Fensters 5 kann das PTC-Element 20 direkt bzw. indirekt auf das Fenstersubstrat 8 aufgebracht werden. Eine thermische Ankopplung und elektrische Isolation kann durch Kleben und/oder Aufpressen erfolgen.

Die in den Fig. 4 und 5 dargestellten Litzen 15 können bei dieser zweiten Variante unmittelbar an das PTC-Element 20 angeschlossen, bspw. angelötet oder über Stiftkontakte, Federkontakte oder ähnliche geeignete flexible Elemente angeschlossen werden. Als Isolationselemente 21 zur Einhaltung der geforderten Luft- und Kriechstrecken kommen thermisch leitende und elektrisch isolierende Materialien in Frage, insbesondere Keramiken (z. Bsp. AL₃O₂) oder Kunststoffe mit Keramikfüllstoffen, bspw. Silikone mit Keramikfüllstoffen zur Verbesserung der Wärmeleitfähigkeit. Zur Optimierung der thermischen Anbindung werden hierbei zwischen die einzelnen Schichten thermisch leitende Materialien eingebracht. Derartige Elemente können auch zum Ausgleichen von ungünstigen Form- oder Maßtoleranzen genutzt werden.

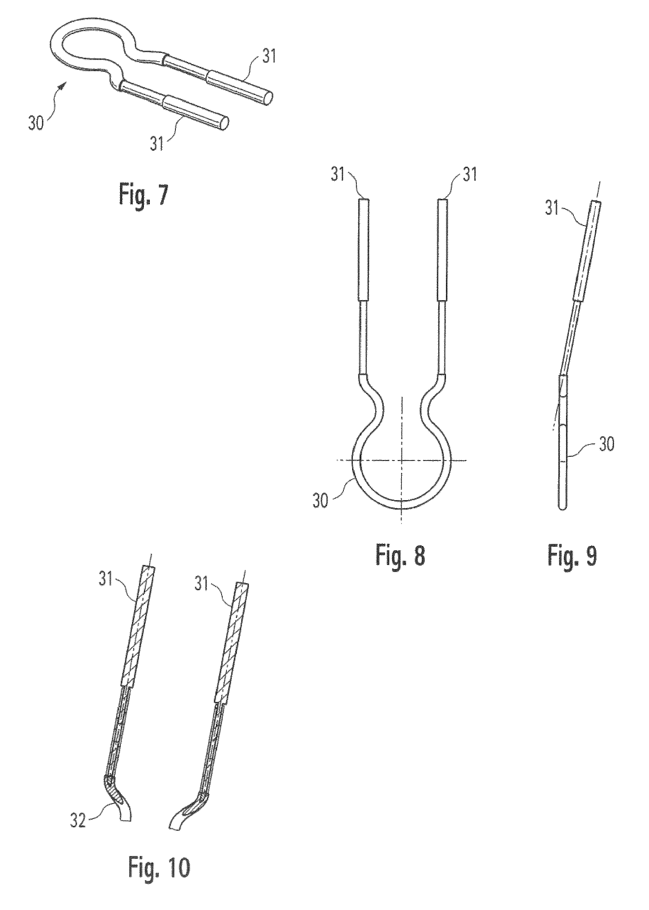

Eine weitere Möglichkeit zur Realisierung einer erfindungsgemäßen Fensterheizung besteht in der Verwendung eines Widerstandsdrahts 30, wie er in den Figuren 7 bis 10 dargestellt ist. Je nach Ausgestaltung des Fensters kann der Widerstandsdraht 30 direkt auf das Fenstersubstrat aufgebracht oder mittels einer oder mehrerer Isolationselemente angebunden werden. Der Widerstandsdraht 30 kann in diesem Fall direkt an zwei stromzufübrenden Litzen 31 angelötet, angecrimpt oder in ähnlicher Weise mit diesen verbunden sein. Auch hier sind spezielle Maßnahmen erforderlich, um die geltenden Normen bezüglich Luft- und Kriechstrecken einzuhalten. Der Widerstandsdraht muss dementsprechend mit einer elektrischen Isolation 32 ummantelt werden, wobei hierzu ein Schlauch, ein Kunststoff- oder Keramikteil verwendet werden könnte. Der schleifenförmig geformte Widerstandsdraht 30 kann dann mit oder ohne Isolation mittels geeigneter Elemente auf das - nicht dargestellte - Fenstersubstrat geklebt oder angepresst werden.

Schließlich bestünde eine weitere Möglichkeit zur Beheizung des Fensters auch noch darin, so genannte Heatpipes einzusetzen, welche für eine Wärmekopplung zwischen Heizquelle und Fenstersubstrat sorgen.

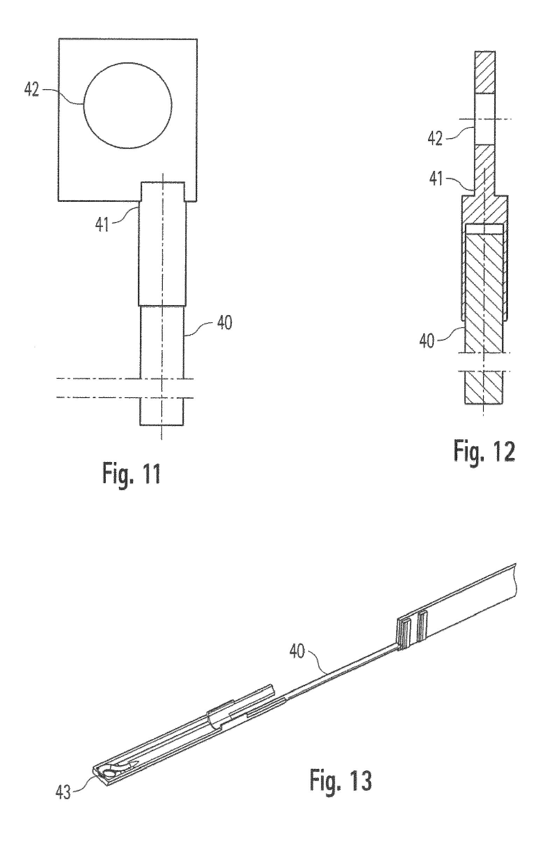

Bei dem in den Figuren 11 und 12 dargestellten Beispiel ist eine an sich bekannte Heatpipe 40 mit einem Zusatzelement in Form eines Wärmetauscherblechs 41 versehen, welches ein Loch 42 aufweist, das im Zentrum des zu beheizenden Fensters liegen kann. Es dient dazu, das Fenster möglichst homogen zu erwärmen. Das Zusatzelement 41 wird hierbei an die Heatpipe angelötet oder auf sonstige Weise mit diesem verbunden. Ein Vorteil dieser Variante besteht darin, dass die eigentliche Wärmequelle, also die Widerstandsheizung und damit die kritischen Bereiche bezüglich Luft- und Kriechstrecken in Bereiche des Handstücks verlagert werden können, welche hinsichtlich des zur Verfügung stehenden Raums weniger kritisch sind. In diesem Bereich erfolgt dann die eigentliche Erwärmung der Heatpipe.

Fig. 13 zeigt schließlich eine zweite Ausführungsform einer Wärmeankopplung mittels einer Heatpipe 40, welche nunmehr wiederum im vorderen Bereich 43, der an dem Fenstersubstrat angeordnet ist, schleifenformig ausgebildet ist.

Letztendlich eröffnet die erfindungsgemäße Lösung die Möglichkeit, das Fenster einer intraoralen Kamera in einfacher und effektiver Weise zu beheizen. Die kompakte Ausgestaltung der erfindungsgemäßen Heizung gewährleistet ferner eine kleine Baugröße des Kamerakopfbereichs, was zu einer verbesserten Handhabung des Geräts führt.

## Patentansprüche

1. Zahnmedizinisches Diagnosegerät mit Lichtquellen zur Illumination eines Zahns, Mitteln zur Bilderfassung des illuminierten Zahns sowie einem Handstück (1), welches ein im Strahlengang der Mittel zur Bilderfassung befindliches Fenster (5) aufweist, wobei ferner Mittel zur Beheizung des Fensters (5) vorgesehen sind, welche durch eine Widerstandsheizung gebildet sind, und
wobei das Handstück (1) ferner mindestens einen Arm (4, 7) mit einem daran angeordneten Lichtaustrittselement aufweist.

2. Zahnmedizinisches Diagnosegerät nach Anspruch 1,
**dadurch gekennzeichnet,**
**dass** der Arm (4, 7) dem Fenster (5) gegenüberliegend angeordnet ist.

3. Zahnmedizinisches Diagnosegerät nach Anspruch 1,
**dadurch gekennzeichnet,**
**dass** das Handstück (1) zwei seitliche Arme (4, 7) aufweist, welche in einem Winkel zu dem Fenster (5) angeordnet sind.

4. Zahnmedizinisches Diagnosegerät nach einem der Ansprüche 1 bis 3,
**dadurch gekennzeichnet,**
**dass** die Widerstandsheizung eine Leiterbahnanordnung (10) aufweist.

5. Zahnmedizinisches Diagnosegerät nach Anspruch 4,
**dadurch gekennzeichnet,**
**dass** die Leiterbahnanordnung (10) im Bereich des Fensters (5) meanderförmig angeordnete Leiterbahnen aufweist.

6. Zahnmedizinisches Diagnosegerät nach Anspruch 4 oder 5,
**dadurch gekennzeichnet,**
**dass** die Leiterbahnanordnung (10) unmittelbar auf einem das Fenster (5) bildenden optisch transparenten Substrat (8) angeordnet ist.

7. Zahnmedizinisches Diagnosegerät nach Anspruch 4 oder 5,
**dadurch gekennzeichnet,**
**dass** die Leiterbahnanordnung (10) auf einem Trägerelement (11) angeordnet ist, welches mit einem das Fenster (5) bildenden optisch transparenten Substrat (8) gekoppelt ist.

8. Zahnmedizinisches Diagnosegerät nach Anspruch 7,
**dadurch gekennzeichnet,**
**dass** auf dem Trägerelement (11) weitere elektronische Bauteile angeordnet sind.

9. Zahnmedizinisches Diagnosegerät nach einem der Ansprüche 4 bis 8,
**dadurch gekennzeichnet,**
**dass** die Mittel zur Beheizung des Fensters (5) ferner Temperaturmesselemente (13) sowie eine Steuereinheit umfassen, welche dazu ausgebildet ist, einen durch die Leiterbahnanordnung (10) fließenden Strom zu regeln.

10. Zahnmedizinisches Diagnosegerät nach einem der Ansprüche 1 bis 3,
**dadurch gekennzeichnet,**
**dass** die Mittel zur Beheizung des Fensters (5) ein PTC-Element (20) umfassen.

11. Zahnmedizinisches Diagnosegerät nach einem der Ansprüche 1 bis 3,
**dadurch gekennzeichnet,**
**dass** die Mittel zur Beheizung des Fensters (5) einen in der Nähe des Fensters (5) befindlichen Widerstandsdraht (30) umfassen.

12. Zahnmedizinisches Diagnosegerät nach einem der vorherigen Ansprüche,
**dadurch gekennzeichnet,**
**dass** die Mittel zur Beheizung des Fensters (5) ummantelt sind.

13. Zahnmedizinisches Diagnosegerät nach einem der Ansprüche 1 bis 3,
**dadurch gekennzeichnet,**
**dass** die Mittel zur Beheizung des Fensters (5) über zumindest eine Heatpipe (40) mit einem das Fenster (5) bildenden optisch transparenten Substrat (8) gekoppelt sind.
